# EUROPEAN PATENT APPLICATION

(11) **EP 4 694 196 A1**
(43) Date of publication of application: **11.02.2026**
(21) Application number: 23930548.5
(22) Date of filing: 30.03.2023
(51) Int. Cl.: H04R 3/00, A61M 21/02, G10K 15/02

(54) **FUNCTIONAL SOUND PRESENTATION SYSTEM, DEVICE, METHOD, AND PROGRAM**

(71) Applicant: IC Co., Ltd., Tokyo 108-6207 (JP); Feat Limited, Tokyo 105-0012 (JP)
(72) Inventor: ARIMITSU, Akihiko, Tokyo 105-0012 (JP)
(74) Representative: Kilian Kilian & Partner mbB
(86) International application number: PCT/JP2023/013234
(87) International publication number: WO 2024/201910

(57) **Abstract**

A functional sound presentation system is provided, including a storage unit that stores element functional sound for coping with symptoms for each symptom, an output specification unit that specifies functional sound to be output based on predetermined setting information or information obtained from a subject, a functional sound generation unit that generates the specified functional sound by synthesizing and/or correcting the element functional sound, and a presentation information generation unit that generates presentation information for presenting the functional sound to the subject.

## Description

### Technical Field

The present invention relates to a device, and the like, that present sound, and the like, to a subject.

### Background Art

In recent years, a system is known that tries to achieve a predetermined purpose by presenting sound in a living space. For example, Patent Literature 1 discloses an invention that tries to control a sensory temperature of human using audio and light control.

### Citation List

### Patent Literature

Patent Literature 1: Japanese Patent Laid-Open No. 2014-129936

### Summary of Invention

### Technical Problem

By the way, in recent years, with the coming of an aged society, various accidents and inconveniences caused by decrease in cognitive functions of elderly people, people who need nursing care, and the like, become problematic. For example, elderly people, and the like, who have difficulty in daily lives due to loss of time sense, impairment of short-term memory, and the like, are increasing, which also leads to degradation of safety and quality of lives of the elderly people.

While various kinds of training, rehabilitation, methods using drugs, and the like, have been proposed to cope with such a problem of decrease in the cognitive functions of elderly people, and the like, they are still under study, and it is desired to propose a potent technique that improves safety or quality of lives of elderly people, and the like.

The present invention has been made in view of the above-described technical background, and an object thereof is to improve safety or quality of a life of a subject.

### Solution to Problem

The above-described technical problem can be solved by a functional sound presentation system, and the like, having the following configuration.

In other words, a functional sound presentation system according to the present invention includes a storage unit that stores element functional sound for coping with symptoms for each symptom, an output specification unit that specifies functional sound to be output based on predetermined setting information or information obtained from a subject, a functional sound generation unit that generates the specified functional sound by synthesizing and/or correcting the element functional sound, and a presentation information generation unit that generates presentation information for presenting the functional sound to the subject.

According to such a configuration, it is possible to present to the subject, functional sound from sound for coping with various kinds of symptoms in accordance with the predetermined setting information or a state of the subject. This makes it possible to improve safety or quality of a life of the subject.

### Advantageous Effect of Invention

According to the present invention, it is possible to improve safety or quality of a life of a subject.

### Brief Description of Drawings

[Figure 1] Figure 1 is an overall configuration diagram of a monitoring support system.
[Figure 2] Figure 2 is a hardware configuration diagram of an information processing device.
[Figure 3] Figure 3 is a hardware configuration diagram of a server device.
[Figure 4] Figure 4 is an explanatory diagram illustrating an example where a sensor device is arranged in a kitchen.
[Figure 5] Figure 5 is an explanatory diagram illustrating an example where the sensor device is installed in a bedroom.
[Figure 6] Figure 6 is an explanatory diagram illustrating an example where the sensor device is attached to a handrail in a corridor.
[Figure 7] Figure 7 is an explanatory diagram illustrating an example where the sensor device is attached to an armed chair placed in a room.
[Figure 8] Figure 8 is an explanatory diagram illustrating another example where the sensor device is installed in the bedroom.
[Figure 9] Figure 9 is an explanatory diagram illustrating an example where the sensor device is attached to a door through which a person moves between rooms.
[Figure 10] Figure 10 is an explanatory diagram illustrating an installation example of an output presentation device in a living space.
[Figure 11] Figure 11 is a functional block diagram of a server device when processing of presenting functional sound, and the like, is performed.
[Figure 12] Figure 12 is a general flowchart regarding operation of a monitoring support system.

### Description of Embodiments

Hereinafter, preferred embodiments of the present invention will be described in detail with reference to the accompanying drawings.

### (1. First Embodiment)

An example will be described as a first embodiment where the present invention is applied to a monitoring support system 100 of elderly people, people who need nursing care, and the like. Note that in the present embodiment, for convenience sake, a person who performs monitoring by utilizing the monitoring support system 100 will be referred to as a monitoring person, and a person who is monitored by the monitoring support system 100 will be referred to as a person to be monitored.

### (1.1 Configuration of Monitoring Support System)

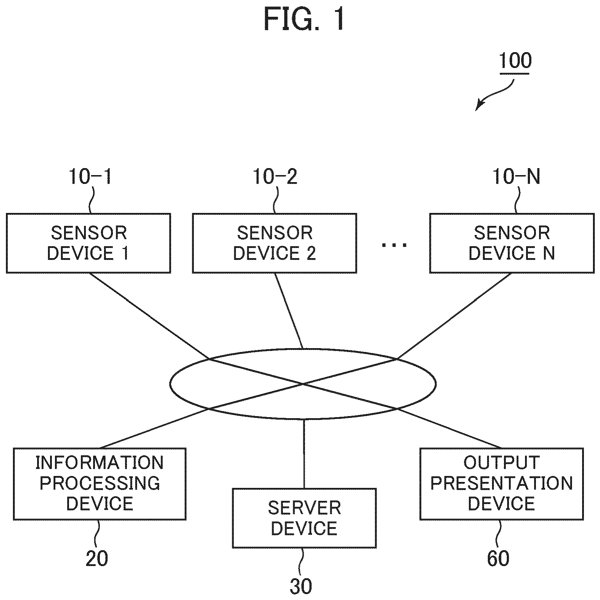

Figure 1 is an overall configuration diagram of the monitoring support system 100 according to the present embodiment. As is clear from the drawing, the monitoring support system 100 includes a plurality of sensor devices 10 (a sensor device 1 (10-1), a sensor device 2 (10-2), ..., a sensor device N (10-N)), an information processing device 20, a server device 30, and an output presentation device 60 and these devices are connected to one another via a network such as the Internet. Note that a configuration of the present network is an example. Further, the server device 30 may be implemented on cloud.

As will be described later, the sensor device 10 is arranged so as to correspond to each room of a living space of the person to be monitored. The sensor device 10 includes a sensor for sensing the person to be monitored, a storage unit such as a ROM and a RAM which stores programs and various kinds of data, a control unit such as a CPU, and a communication unit including a communication unit for providing and receiving information to and from an external device. Information detected at the sensor of the sensor device 10 is transmitted to other devices, for example, the server device 30 via a network.

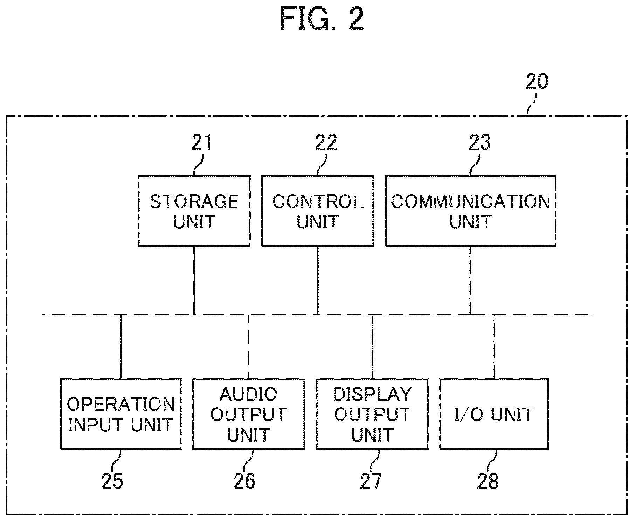

Figure 2 is a hardware configuration diagram of the information processing device 20 according to the present embodiment. As is clear from the drawing, the information processing device 20 includes a storage unit 21, a control unit 22, a communication unit 23, an operation input unit 25, an audio output unit 26, a display output unit 27 and an I/O unit 28, and these are connected to one another via a bus, and the like.

The storage unit 21, which is a storage unit such as a ROM/RAM, a hard disk and a flash memory, stores various kinds of programs or data for executing operation which will be described later. The control unit 22, which is a control device such as a CPU, executes programs to implement various kinds of operation which will be described later. The communication unit 23 is a communication unit for providing and receiving information to and from an external device via a network. The operation input unit 25 provides input information detected via an input device such as a mouse, a keyboard and a touch panel to the control unit 22. The audio output unit 26 performs audio output to an output device such as a connected speaker in accordance with control by the control unit 22. The display output unit 27 outputs image information to a display output device such as a display in accordance with control by the control unit 22. The I/O unit 28 performs input/output processing to/from an external device.

The information processing device 20 provides/receives information to/from the sensor device 10 or the server device 30. The monitoring person or a person who introduces the system can perform various kinds of setting processing by operating the information processing device 20. For example, the installed sensor devices 10 can be associated with rooms, and the like, in the living space on the monitoring support system 100. Further, various kinds of information can be presented to the monitoring person via the information processing device 20. For example, a state of the person to be monitored, and the like, can be presented to the monitoring person.

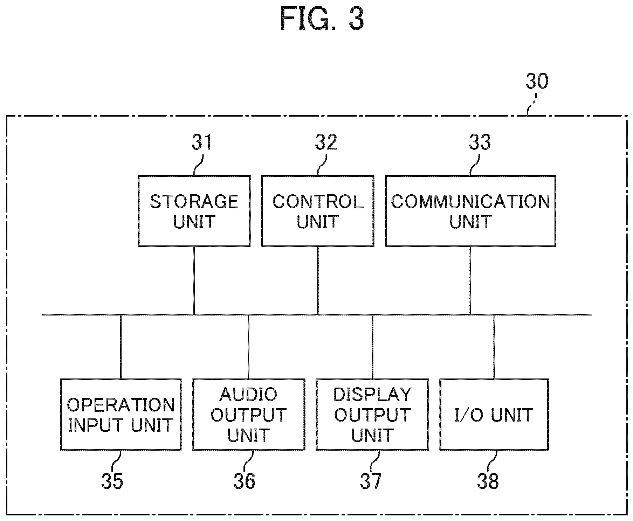

Figure 3 is a hardware configuration diagram of the server device 30 according to the present embodiment. As is clear from the drawing, the server device 30 includes a storage unit 31, a control unit 32, a communication unit 33, an operation input unit 35, an audio output unit 36, a display output unit 37, and an I/O unit 38, and these are connected to one another via a bus, and the like.

The storage unit 31, which is a storage unit such as a ROM/RAM, a hard disk and a flash memory, stores various kinds of programs or data for executing operation which will be described later. The control unit 32, which is a control device such as a CPU and a GPU, executes programs to implement various kinds of operation which will be described later. The communication unit 33 is a communication unit for providing/receiving information to/from an external device via a network. The operation input unit 35 provides input information detected via an input device such as a mouse, a keyboard and a touch panel to the control unit 32. The audio output unit 36 performs audio output to an output device such as a connected speaker in accordance with control by the control unit 32. The display output unit 37 outputs image information to a display output device such as a display in accordance with control by the control unit 32. The I/O unit 38 performs input/output processing to/from an external device.

The server device 30 provides and receives information to and from the sensor device 10, the information processing device 20 or the output presentation device 60 and executes various kinds of processing which will be described later.

The output presentation device 60 includes a presentation unit including a speaker, a light source and a vibration unit, a storage unit which is a ROM, a RAM, or the like, and stores programs and various kinds of data, a control unit such as a CPU, and a communication unit including a communication unit for providing and receiving information to and from an external device.

The output presentation device 60 is installed in the living space of the person to be monitored and presents presentation information received from the server device 30 to the person to be monitored. For example, in a case where presentation information regarding functional sound is received from the server device 30, the functional sound is presented to the person to be monitored via the speaker. Note that the output presentation device 60 and the sensor device 10 installed in the same living space may be integrally constituted.

The sensor device 10 is attached to various portions within the living space of the person to be monitored. The portions are, for example, a floor and a wall of the room, a handrail, home electrical appliance, furniture, and the like.

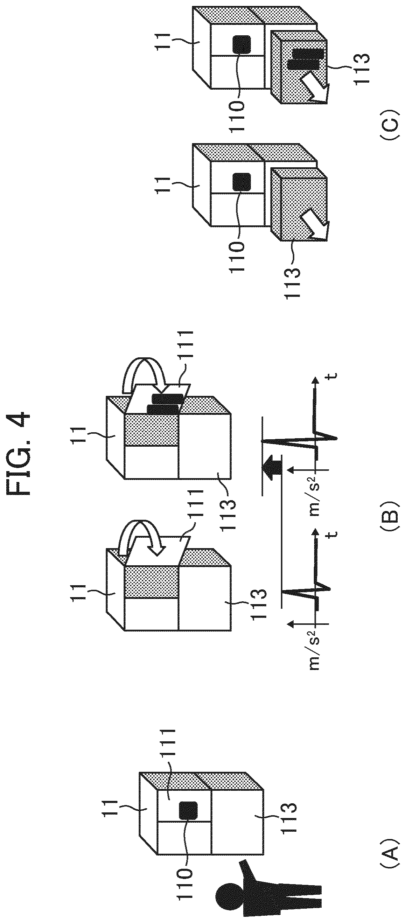

Figure 4 is an explanatory diagram illustrating an example where the sensor device 10 is attached to a refrigerator 11 arranged in a kitchen. As is clear from the drawing, the refrigerator 11 has a two-stage configuration including an upper part and a lower part, a pair of doors 111 is arranged in the upper part, and a slid-out storage 113 is arranged in the lower part. (A) of Figure 4 illustrates an overall configuration of the refrigerator when the doors are closed. (B) of Figure 4 is an explanatory diagram regarding opening and closing of the pair of doors 111 in the upper part. (C) of Figure 4 is an explanatory diagram regarding opening and closing of the storage 113 in the lower part.

As is clear from (A) of Figure 4, an acceleration sensor 110 as the sensor of the sensor device 10 is provided at each door of the pair of doors 111. Further, an acceleration sensor (not illustrated) as the sensor of the sensor device 10 is also provided at the storage 113 in the lower part.

As is clear from (B) of Figure 4, the acceleration sensor 110 is provided at each door of the pair of doors 111. Opening and closing of the door can be detected from a time-series signal obtained at this acceleration sensor 110. Further, if a container of drink, or the like, is stored inside the door, the door becomes heavy. As is clear from left and right drawings of (B) of Figure 4, this change of weight can be detected as a change of the detection value at the acceleration sensor 110, and thus, whether or not there is a container inside the door, and its interior content are also indirectly detected.

As is clear from (C) of Figure 4, the acceleration sensor (not illustrated) is provided at the storage 113, and opening and closing of the storage 113 can be detected from a time-series signal obtained at this acceleration sensor. Further, if a container of drink, or the like, is stored in the storage 113, a weight of the storage 113 becomes heavy. As is clear from left and right drawings of (C) of Figure 4, this change of the weight can be detected as a change of the detection value at the acceleration sensor, and thus, whether or not there is a container inside the storage 113 and its interior content are also indirectly detected.

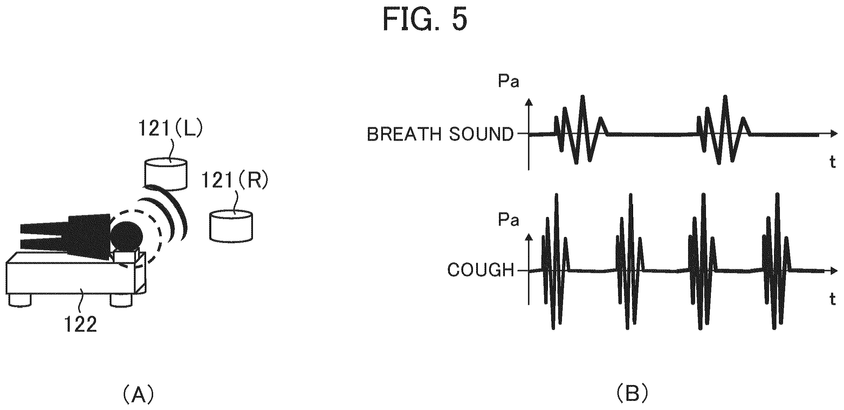

Figure 5 is an explanatory diagram illustrating an example where the sensor device 10 is installed in a bedroom. (A) of Figure 5 is a view illustrating an arrangement configuration example, and (B) of Figure 5 is an explanatory diagram regarding sound collection examples. As is clear from (A) of Figure 5, a pair of left and right microphones, that is, a microphone 121(L) and a microphone 121(R) are arranged around a bed 122 in the bedroom as the sensor of the sensor device 10. These microphones collect sound generated from the person to be monitored who is sleeping on the bed 122 in chronological order. For example, as indicated in (B) of Figure 5, breath sound and cough of the person to be monitored can be detected from sound pressure waveforms obtained by collecting sound thereof. Note that, in detection, sound in a target area (around the bed 122) may be collected by beamforming, or various kinds of publicly known signal processing such as removal of noise other than target sound may be performed.

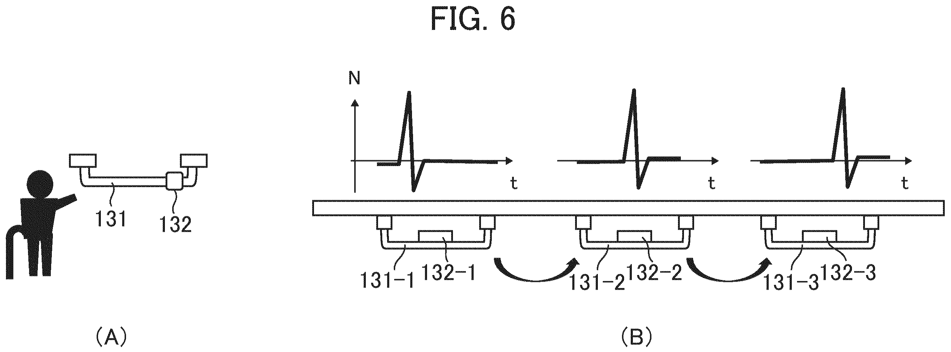

Figure 6 is an explanatory diagram illustrating an example where the sensor device 10 is attached to a handrail in a corridor. As illustrated in (A) of Figure 6, a strain sensor 132 is provided at a rod-like handrail 131 as the sensor of the sensor device 10. Load, torsion moment, and the like, applied to the handrail can be detected from a time-series signal obtained from this strain sensor 132. A state of the person to be monitored, for example, whether or not he/she has physically disabled arms or legs can be detected by detecting these.

Further, a strain sensor may be provided at each of a plurality of handrails. In (B) of Figure 6, three handrails 131 (131-1, 131-2, 131-3) are continuously arranged, and strain sensors 132 (132-1, 132-2, 132-3) are respectively attached to the handrails 131. According to such a configuration including a plurality of sensors, not only load and torsion moment can be detected at each strain sensor 132, but a moving speed of the person to be monitored who moves while holding on the handrails can be also calculated by analyzing time-series signals obtained from the respective strain sensors 132 in an integrated manner.

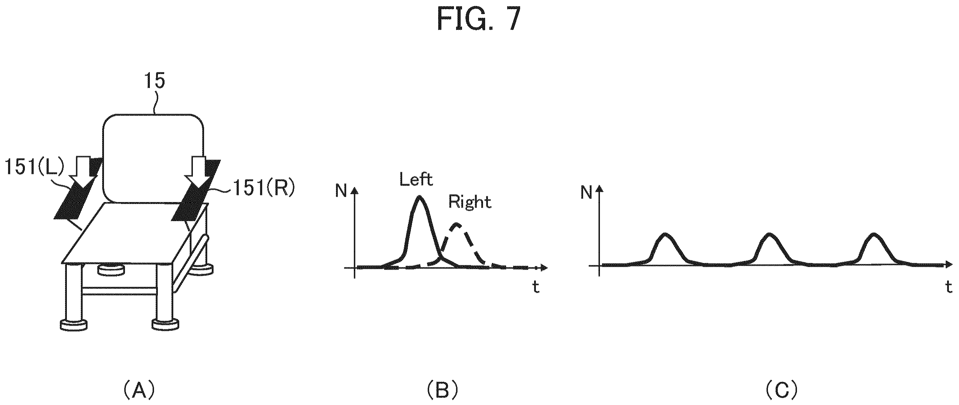

Figure 7 is an explanatory diagram illustrating an example where the sensor device 10 is attached to an armed chair placed in a room. As illustrated in (A) of Figure 7, a pair of strain sensors 151 (151(L), 151(R)) is attached to left and right armrests of an armed chair 15 as the sensor of the sensor device 10. Various kinds of information can be detected from time-series signals of load, vibration, strain, and the like, detected at this pair of strain sensors 151.

(B) of Figure 7 is an example of signals detected at the pair of strain sensors 151. A magnitude and a timing of detected load can be estimated from such signals, and a dominant hand, walking ability, a posture, breathing, and the like, of the person to be monitored can be estimated from a difference between signals from the left and right strain sensors, and the like.

(C) of Figure 7 is another example of the signal detected at the pair of strain sensors 151. A state of breathing, or the like, of the person to be monitored can be estimated from such a periodic signal.

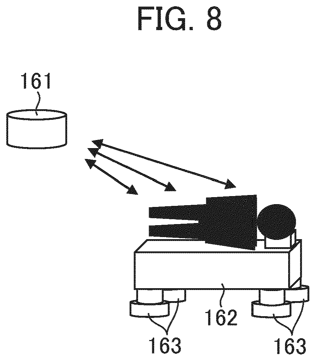

Figure 8 is an explanatory diagram illustrating another example where the sensor device 10 is installed in the bedroom. In the example of Figure 8, a strain sensor 163 is arranged on a back side of each of four legs of a bed 162 as the sensor of the sensor device 10. For example, a state of the person to be monitored on the bed 162 can be estimated from time-series signals obtained from the strain sensors 163. Further, a distance sensor 161 is arranged at a position a predetermined distance away from the bed 162 as the sensor of the sensor device 10. For example, behavior of the person to be monitored can be detected from a time-series signal obtained from this distance sensor 161.

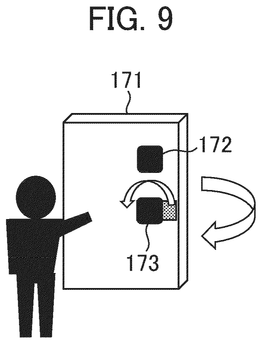

Figure 9 is an explanatory diagram illustrating an example where the sensor device 10 is attached to a door through which the person to be monitored moves between rooms. As is clear from Figure 9, a door 171 is configured to be able to be open and closed by a knob being pushed/pulled while being rotated, and acceleration sensors 172, 173 are attached to the door 171 and the knob. Whether the door is open or closed, an opening/closing speed, and the like, can be detected from a time-series signal obtained from this acceleration sensor 172. Note that a frequency of movement between rooms and action performed in the room, and the like, can be calculated through these.

While attachment examples of the sensor device 10 have been described above, all examples are merely illustrative, and the present invention is not limited to such configurations. Thus, to detect living conditions of the person to be monitored, any sensor may be used, and attachment portions can be modified in various manners.

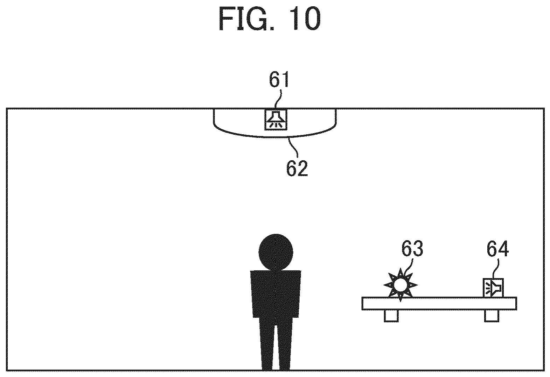

Figure 10 is an explanatory diagram illustrating an installation example of the output presentation device 60 in the living space. As is clear from the example in Figure 10, a first speaker 61 and a light source 62 are installed on a ceiling of the room as presentation units of the output presentation device 60. Further, a second speaker 64 and a vibration unit 63 are installed on a handrail. A desired auditory, visual or haptic stimulus is presented to the person to be monitored via these presentation units. Note that the control unit and other components of the output presentation device 60 are arranged inside or outside the room (not illustrated).

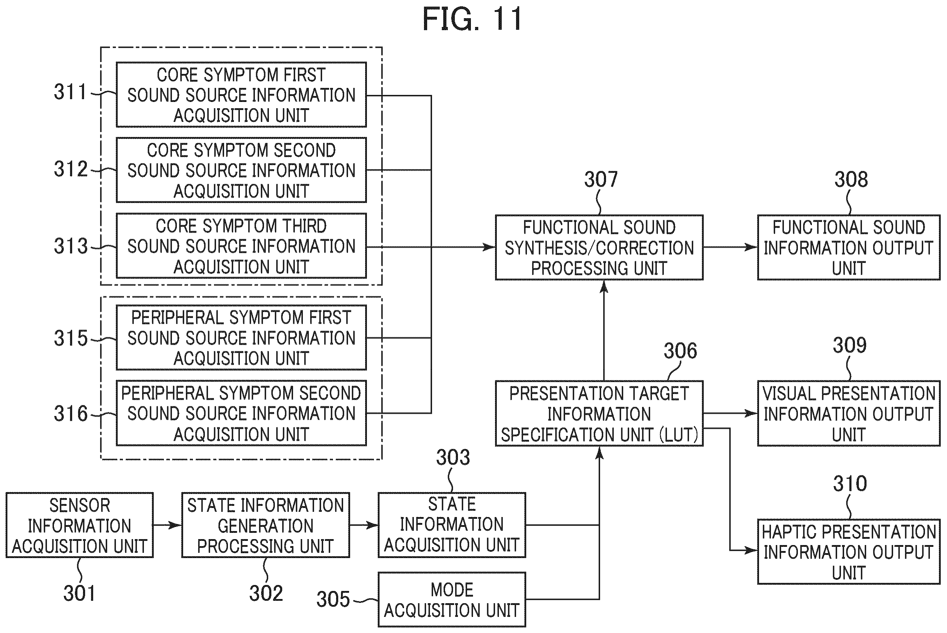

Figure 11 is a functional block diagram of the server device 30 when processing of presenting functional sound, and the like, is performed. As is clear from Figure 11, a sensor information acquisition unit 301 acquires sensor information detected at the sensor device 10 from the storage unit 31 of the server device 30 and provides the sensor information to a state information generation processing unit 302.

The state information generation processing unit 302 performs predetermined signal processing based on the sensor information and generates information for estimating a state such as a health state of the person to be monitored, that is, state information. The state information acquisition unit 303 acquires this state information and provides the state information to a presentation target information specification unit 306.

The presentation target information specification unit 306 specifies functional sound, visual presentation information and haptic presentation information corresponding to the state information using a predetermined look-up table (LUT).

A functional sound synthesis/correction processing unit 307 synthesizes and/or corrects sound related to one or more sound sources to obtain specified functional sound. Here, each sound source is acquired from the storage unit 31 by a sound source information acquisition unit (311 to 316) provided for each symptom.

More specifically, the respective sound sources to be acquired are first roughly classified into a sound source for core symptom and a sound source for peripheral symptom. The core symptom represents a core symptom among symptoms of the person to be monitored and is, for example, a symptom such as loss of time sense, impairment of short-term memory, decrease in a cognitive function. Further, the peripheral symptom represents a peripheral symptom other than the core symptom among the symptoms of the person to be monitored and is, for example, decrease in various kinds of functions (such as decrease in hearing ability and loss in muscle strength), problems regarding specific action (for example, problems regarding eating (such as loss of appetite and overeating), problems regarding going out (such as wandering)), or the like.

A core symptom first sound source information acquisition unit 311 performs processing of reading and acquiring from the storage unit 31, a sound source for core symptom, particularly, a sound source regarding sound that provides awareness regarding periodicity to cope with loss of time sense. Note that the periodicity is, for example, flow of time of one day, day of the week, four seasons, or the like. The sound that provides awareness regarding periodicity invokes time sense to the subject by components of sound, for example, a frequency, an interval of the sound, a sound pressure, clarity, melody (tune), and the like, changing. Note that sound of a specific motif such as sound of sand dropping in an hourglass may be used.

A core symptom second sound source information acquisition unit 312 performs processing of reading and acquiring from the storage unit 31, a sound source for core symptom, particularly, a sound source regarding sound that provides awareness regarding an event to cope with impairment of short-term memory. The event is, for example, taking medicine, having a meal, taking a shower, going to a bathroom, engaging in a mobility activity, or the like. The sound that provides awareness regarding these events is, for example, collision sound of tablets or sound of opening/closing a capsule in a case where it is time to take medicine, cooking sound or sound of dishes (collision sound of metals) in a case where it is time to have a meal, sound of running water in a case where it is time to take a shower or go to a bathroom, walking sound in a case where it is time to engage in a mobility activity, or the like.

According to such a configuration, the event is associated with sound related to content of the event, so that it is possible to provide awareness regarding the event to the subject through the functional sound.

A core symptom third sound source acquisition unit 313 performs processing of reading and acquiring from the storage unit 31, a sound source for core symptom, particularly, a sound source regarding sound for coping with decrease in a cognitive function. The sound for coping with decrease in the cognitive function is, for example, sound (or warning sound) that provides awareness regarding a surrounding environmental object (such as a step), or the like.

A peripheral symptom first sound source information acquisition unit 315 performs processing of reading and acquiring from the storage unit 31, a sound source for peripheral symptom, particularly, a sound source regarding sound that provides awareness of decrease in a function to cope with decrease in a physical function. A peripheral symptom second sound source information acquisition unit 316 performs processing of reading and acquiring from the storage unit 31, a sound source for peripheral symptom, particularly, a sound source regarding sound that encourages or suppresses specific action to cope with a problem of specific action (for example, having a meal or going out).

In this manner, the sound related to each sound source is configured to be able to cope with a specific symptom and, for example, components constituting the sound change over time. This change includes, for example, changes regarding pitch of the sound (frequency), loudness (sound pressure), timbre (frequency component), musicality (such as melody (tune), harmony and rhythm).

According to such a configuration, the person to be monitored can obtain awareness regarding time or an event or can be encouraged or suppressed to perform specific action through change of the components of the presented sound.

A functional sound information output unit 308 performs processing of acquiring and outputting the synthesized or corrected functional sound. This functional sound is presented to the person to be monitored via the output presentation device 60.

A visual presentation information output unit 309 performs processing of acquiring and outputting the visual presentation information specified at the presentation target information specification unit 306. This visual presentation information is presented to the person to be monitored via the output presentation device 60.

A haptic presentation information output unit 310 performs processing of acquiring and outputting the haptic presentation information specified at the presentation target information specification unit 306. This haptic presentation information is presented to the person to be monitored via the output presentation device 60.

Note that the presentation target information specification unit 306 can specify the functional sound, the visual presentation information and the haptic presentation information not only from the state information but in accordance with a predetermined mode acquired from the storage unit 31 by a mode acquisition unit 305. This mode may be set in advance by the monitoring person, or the like, through the information processing device 20, or the like.

According to such a configuration, it is possible to easily specify the functional sound, and the like, by setting a mode.

### (1.2 Operation of Monitoring Support System)

Operation of the monitoring support system 100 will be described next.

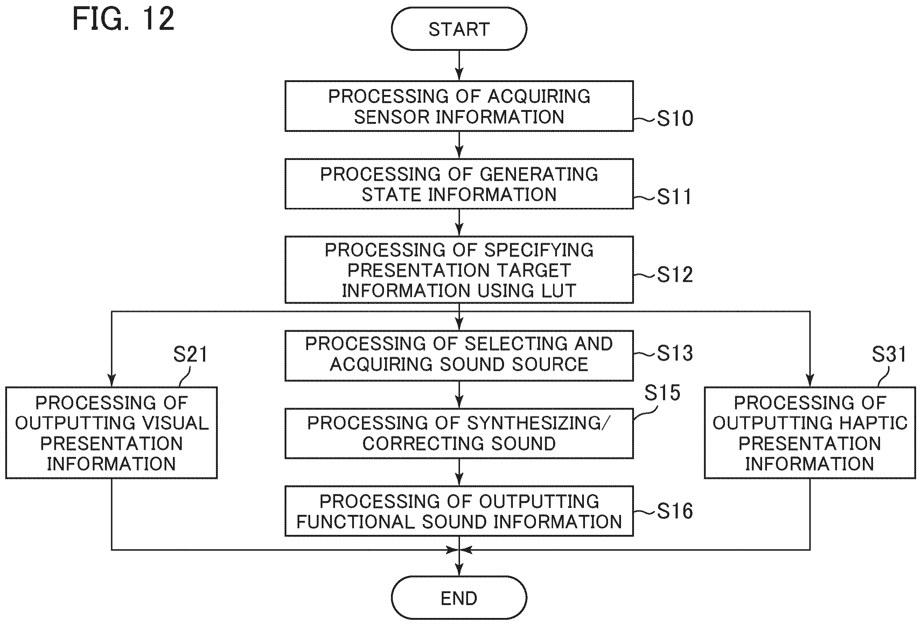

Figure 12 is a general flowchart regarding the operation of the monitoring support system 100. As is clear from Figure 12, when processing starts, the sensor information acquisition unit 301 performs processing of acquiring the time-series sensor information detected at the sensor device 10 and providing the time-series sensor information to the state information generation processing unit 302 (S10).

The state information generation processing unit 302 performs predetermined information processing on the acquired sensor information to generate information regarding the state of the person to be monitored (S11). The predetermined information processing is, for example, processing of calculating each feature amount for each sensor based on sensor information transformed into a signal in a frequency domain or the sensor information as is in a time domain, normalizing and clustering the feature amount and performing factor analysis on a result of the clustering processing. The state of the person to be monitored may be estimated from the one or more factors or a change in a time direction of the factor.

According to such a configuration, it is possible to accurately classify the state of the subject while reducing influence of sensor noise, and the like, by using clustering processing.

Note that a clustering model may be generated by a machine learning algorithm such as DBSCAN.

According to such a configuration, it is possible to further accurately classify the state of the subject by the machine learning technique.

In the present embodiment, the factor may include a mobility factor that is a factor regarding mobility of the person to be monitored, an eating factor that is a factor regarding eating of the person to be monitored, and a hygiene factor that is a factor regarding hygiene of the person to be monitored. Note that the factor may include a sleeping factor that is a factor regarding sleeping.

Note that the information processing is not limited to such a method. Any processing may be used if the processing is capable of evaluating the state of the person to be monitored from the sensor information.

According to such a configuration, it is possible to present functional sound appropriate for the state of the person to be monitored by sensing a situation in the living space of the person to be monitored.

After the processing of generating the state information, the state information acquisition unit 303 provides the state information to the presentation target information specification unit 306.

The presentation target information specification unit 306 that has acquired the state information performs processing of specifying information to be presented using a look-up table (LUT) (S12). More specifically, the LUT stores a relationship between the state information, and the functional sound, the visual presentation information and the haptic stimulus information corresponding to the state information. Thus, the presentation target information specification unit 306 can specify the corresponding functional sound, visual presentation information and haptic stimulus information from the LUT based on the state information.

According to such a configuration, it is possible to specify the functional sound, and the like, to be output rapidly by the look-up table (LUT).

After the specification processing, the function sound synthesis/correction processing unit 307 performs processing of acquiring sound sources necessary for generating the specified functional sound from the respective sound source information acquisition units (311 to 316) (S13). After this acquisition processing, the functional sound synthesis/correction processing unit 307 performs processing of synthesizing and/or correcting sound related to the sound source for core symptom and/or the sound source for peripheral symptom to generate target functional sound specified at the presentation target information specification unit 306 (S15).

According to such a configuration, the functional sound can be generated based on sound respectively corresponding to the core symptom and the peripheral symptom, so that effective presentation of the functional sound can be implemented.

After the processing of generating the functional sound, the functional sound information output unit 308 performs processing of outputting functional sound information (S16). More specifically, the functional sound information output unit 308 performs processing of transmitting the functional sound information to the output presentation device 60 so as to perform output from a speaker of the output presentation device 60. By this means, the person to be monitored receives presentation of the functional sound from the speaker installed in the living space.

According to such a configuration, it is possible to provide awareness regarding time or an event to the person to be monitored having a symptom such as loss of time sense, impairment of short-term memory or decrease in a cognitive function or encourage or suppress action through the functional sound so that it is possible to improve safety or quality of a life of the subject.

After the processing of specifying the presentation target information, the visual presentation information output unit 309 performs processing of outputting the specified visual presentation information (S21). More specifically, the visual presentation information output unit 309 performs processing of transmitting the visual presentation information, for example, brightness and color of the light source, a light emission pattern, and the like, to the output presentation device 60 so as to perform presentation at the light source of the output presentation device 60. By this means, the person to be monitored receives presentation of functional visual presentation information from the light source installed in the living space.

According to such a configuration, it is possible to further improve safety or quality of a life of the person to be monitored by performing presentation in conjunction with a visual stimulus to the person to be monitored (complex stimulation).

Further, after the processing of specifying the presentation target information, the haptic presentation information output unit 310 performs processing of outputting the specified haptic presentation information (S31). More specifically, the haptic presentation information output unit 310 performs processing of transmitting the haptic presentation information, for example, a vibration pattern at the vibration unit to the output presentation device 60 so as to perform presentation at the vibration unit of the output presentation device 60. By this means, the person to be monitored receives presentation of functional haptic presentation information from the vibration unit installed in the living space.

According to such a configuration, it is possible to further improve safety or quality of a life of the person to be monitored by performing presentation in conjunction with a haptic stimulus (such as vibration) to the person to be monitored (complex stimulation).

If these kinds of processing are completed, the processing ends.

According to the configuration described above, it is possible to present to the person to be monitored, the functional sound, and the like, from sound for coping with various kinds of symptoms in accordance with the predetermined setting information or the state of the subject. This makes it possible to improve safety or quality of a life of the subject.

### (2. Modifications)

The present invention can be implemented while being modified in various manners.

While in the above-described embodiment, the present invention is applied to a monitoring support system of elderly people, and the like, a target to which the present invention is applied is not limited to such a system. Thus, for example, the present invention is applicable to any system that requires presentation of functional sound, and the like.

While in the above-described embodiment, description has been provided assuming that the presentation unit of the output presentation device 60 performs auditory, visual or haptic presentation, the present invention is not limited to such a configuration. Thus, the presentation unit may perform presentation to other senses among five senses.

While in the above-described embodiment, a configuration in which presentation of the functional sound, and the like, is controlled has been described, it is also possible to employ a configuration in which presentation is controlled in conjunction with a timing of presentation of the functional sound.

In this case, the LUT of the presentation target information specification unit 306 stores presentation timing information along with the functional sound, and the like, and the presentation target information specification unit 306 specifies the presentation timing information along with the functional sound, and the like. A presentation timing control unit that is a functional block of the server device 30 acquires the presentation timing information along with the functional sound, and the like, and provides information related to the functional sound, and the like, to the respective output units (308 to 310) based on the presentation timing related to the presentation timing information. By this means, the person to be monitored receives presentation of the functional sound, and the like, at a predetermined timing. For example, by presenting the functional sound, for example, indicating morning, noon and night to the person to be monitored who has lost time sense at predetermined time of morning, noon and night, it is possible to invoke the time sense of the person to be monitored.

According to such a configuration, it is possible to more effectively present the functional sound, and the like, to the person to be monitored by controlling the presentation timing of the functional sound, and the like.

While the embodiments of the present invention have been described above, the above-described embodiments merely indicate part of application examples of the present invention, and are not intended to limit the technical scope of the present invention to specific configurations of the above-described embodiments. Further, the above-described embodiments can be combined as appropriate within a range not causing inconsistency.

### Industrial Applicability

The present invention can be utilized in industries that manufacture a sound presentation device, and the like.

### Reference Signs List

- 10: Sensor device
- 20: Information processing device
- 30: Server device
- 60: Output presentation device
- 100: Monitoring support system

## Claims

1. A functional sound presentation system comprising:
a storage unit that stores element functional sound for coping with symptoms for each symptom;
an output specification unit that specifies functional sound to be output based on predetermined setting information or information obtained from a subject;
a functional sound generation unit that generates the specified functional sound by synthesizing and/or correcting the element functional sound; and
a presentation information generation unit that generates presentation information for presenting the functional sound to the subject.

2. The functional sound presentation system according to claim 1, wherein the element functional sound is related to sound that provides awareness regarding time or an event to the subject or sound that encourages or suppresses specific action of the subject.

3. The functional sound presentation system according to claim 2, wherein elements constituting the sound that provides awareness regarding the time or the event or the sound that encourages or suppresses the specific action change over time.

4. The functional sound presentation system according to claim 2, wherein the sound that provides awareness regarding the event includes sound related to content of the event.

5. The functional sound presentation system according to claim 1, wherein the symptoms further include symptoms belonging to a core symptom and symptoms belonging to a peripheral symptom.

6. The functional sound presentation system according to claim 1, wherein the information obtained from the subject is generated based on sensor detection information obtained via a sensor provided in a living space of the subject.

7. The functional sound presentation system according to claim 1, wherein
the output specification unit further specifies a visual stimulation pattern to be output based on the predetermined setting information or the information obtained from the subject, and
the presentation information generation unit further generates information for presenting the specified visual stimulation pattern.

8. The functional sound presentation system according to claim 1, wherein
the output specification unit further specifies a haptic presentation pattern to be output based on the predetermined setting information or the information obtained from the subject, and
the presentation information generation unit further generates information for presenting the specified haptic presentation pattern.

9. The functional sound presentation system according to claim 1, wherein the output specification unit specifies functional sound to be output from the predetermined setting information or the information obtained from the subject using a look-up table.

10. The functional sound presentation system according to claim 1, wherein the setting information is predetermined mode information.

11. The functional sound presentation system according to claim 1, wherein
the output specification unit further specifies a presentation timing of the functional sound based on the predetermined setting information or the information obtained from the subject, and
the presentation information generation unit generates the presentation information based on the presentation timing.

12. A functional sound presentation device comprising:
a storage unit that stores element functional sound for coping with symptoms for each symptom;
an output specification unit that specifies functional sound to be output based on predetermined setting information or information obtained from a subject;
a functional sound generation unit that generates the specified functional sound by synthesizing and/or correcting the element functional sound; and
a presentation information generation unit that generates presentation information for presenting the functional sound to the subject.

13. A functional sound presentation method comprising:
a storage step of storing element functional sound for coping with symptoms for each symptom;
an output specification step of specifying functional sound to be output based on predetermined setting information or information obtained from a subject;
a functional sound generation step of generating the specified functional sound by synthesizing and/or correcting the element functional sound; and
a presentation information generation step of generating presentation information for presenting the functional sound to the subject.

14. A functional sound presentation program comprising:
a storage step of storing element functional sound for coping with symptoms for each symptom;
an output specification step of specifying functional sound to be output based on predetermined setting information or information obtained from a subject;
a functional sound generation step of generating the specified functional sound by synthesizing and/or correcting the element functional sound; and
a presentation information generation step of generating presentation information for presenting the functional sound to the subject.
